# EUROPÄISCHE PATENTSCHRIFT

(11) **EP 0 513 947 B1**
(45) Veröffentlichungstag und Bekanntmachung des Hinweises auf die Patenterteilung: **15.11.1995**
(21) Anmeldenummer: 92250103.6
(22) Anmeldetag: 30.04.1992
(51) Int. Cl.: C07J 53/00, A61K 31/565

(54) **14Alpha,16alpha-Ethano- und 14alpha,16alpha-Ethenoestratriene**
14 Alpha, 16alpha-ethano- and 14alpha, 16alpha ethenoestratrienes
14Alpha, 16alpha-ethano- et 14alpha, 16alpha-etheno-estratriènes

(30) Priorität: 30.04.1991 DE 4114635
(43) Veröffentlichungstag der Anmeldung: 19.11.1992
(73) Patentinhaber: SCHERING AKTIENGESELLSCHAFT, 13342 Berlin (DE)
(72) Erfinder: Laurent, Henry, Dr., W-1000 Berlin 28 (DE); Esperling, Peter, W-1000 Berlin 42 (DE); Elger, Walter, Dr., W-1000 Berlin 33 (DE); Krattenmacher, Rolf, Dr., W-1000 Berlin 44 (DE)

(56) Entgegenhaltungen:
- EP-A- 0 372 665
- WO-A-88/01275
- DE-A- 3 808 679

## Beschreibung

Die Erfindung betrifft 14α,16α-Ethano- und 14α,16α-Etheno-estratriene der allgemeinen Formel **I**, 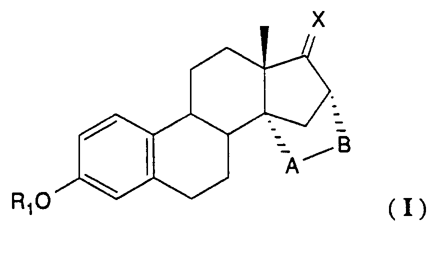
worin
- A - B: eine C-C-Einfach- oder C-C-Doppelbindung,
- R₁: ein Wasserstoffatom, eine Methyl- oder Acylgruppe mit 1 - 12 Kohlenstoffatomen und
- X: Sauerstoff oder 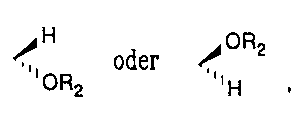 wobei R₂ ein Wasserstoffatom oder eine Acylgruppe mit 1 - 12 Kohlenstoffatomen darstellt,
bedeuten,
ein Verfahren zu ihrer Herstellung, pharmazeutische Präparate, die diese Verbindungen enthalten, sowie deren Verwendung zur Herstellung von Arzneimitteln.

Als Acylgruppen R₁ und R₂ kommen Reste von organischen Carbonsäuren mit 1 - 12 Kohlenstoffatomen in Frage. Sie leiten sich ab von aliphatischen, cycloaliphatischen, aliphatisch-cycloaliphatischen, cycloaliphatisch-aliphatischen und aromatischen Monocarbonsäuren mit 1 - 12 Kohlenstoffatomen. Die Anzahl der Kohlenstoffatome im Ring variiert von 3 - 5. Bevorzugt werden als Reste R₁ und R₂ die Acylgruppen der Essig-, Propion-, Butter-, Isobutter-, Pivalin-, Capron-, Heptyl-, Capryl-, Pelargon-, Decan-, Undecan-, Dodecan-, 3-Cyclopentylpropion- und Benzoesäure. Insbesondere werden die Acylreste von Carbonsäuren mit 2 - 7 Kohlenstoffatomen bevorzugt.

Die neuen 14α,16α-Ethano- und 14α,16α-Etheno-estratriene sind sehr starke Estrogene. Im Allen-Doisy-Test sind die Verbindungen der allgemeinen Formel **I** starker wirksam als Estradiol, insbesondere nach oraler Applikation. Überraschend ist vor allem die weitaus bessere Wirkung der erfindungsgemäßen 17α-Hydroxyverbindungen im Vergleich zum natürlich vorkommenden Estradiol mit 17α-Hydroxygruppe (17α-Estradiol), wie aus der Tabelle 1 hervorgeht.

**Tabelle 1**

| **Verbindung** | Allen-Doisy-Test s.c. µg/Tier/Tag [ED₅₀] | Allen-Doisy-Test p.o. µg/Tier/Tag [ED₅₀] | Estrogen-Rezeptor-Test [KF] |
|---|---|---|---|
| Estradiol | 0.2-0.5 | 50-100 | 1.0 |
| 17α-Estradiol | 100 | > 1000 | 46.0 |
| 14α,16α-Ethano-estra-1,3,5(10)-trien-3,17α-diol | 0.1 - 0.3 | 3 - 10 | 0.5 |
| 14α,16α-Etheno-estra-1,3,5(10)-trien-3,17α-diol | | | 1.5 |
| 14α,16α-Ethano-estra-1,3,5(10)-trien-3,17β-diol | 0.1 - 0.3 | 1.0 | 1.0 |

Im Allen-Doisy-Test wird eine Bewertung von Vaginalabstrichen bei ovariektomierten Ratten an den Tagen 3 - 5 (d3 - d5) nach der einmaligen Applikation am Tag 1 (d1) der Prüfsubstanz vorgenommen. Folgende Zyklusstadien werden unterschieden:
- 1 =: Diöstrus (Leukozyten und kernhaltige Epithelzellen),
- 2 =: Proöstrus (kernhaltige Epithelzellen),
- 3 =: Östrus (kernlose Hornschollen),
- 4 =: Metöstrus (kernlose Hornschollen, Leukozyten, Epithelzellen).

Estrogen wirksame Substanzen führen nach oraler oder subcutaner Applikation zur Proliferation des Vaginalepithels und zur Verhornung der oberflächlichen Zell-Lagen. Als Schwellenwert wird diejenige Menge eines Estrogens angesehen, bei der 50 % der Tiere Stadium 3 erreichen. Außerdem bewirken Estrogene eine Zunahme des Uterusgewichts.

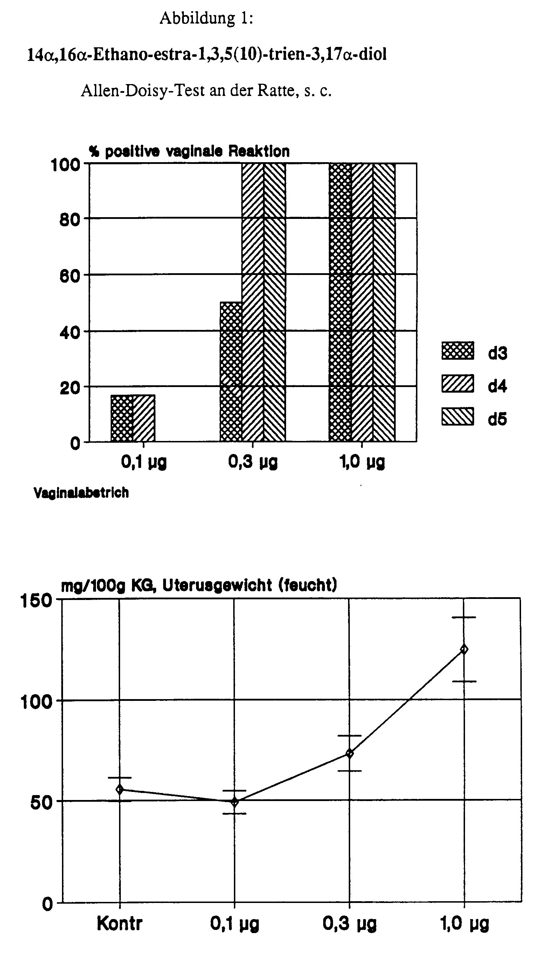

Am Beispiel des erfindungsgemäßen 14α,16α-Ethano-estra-1,3,5(10)-trien-3,17α-diols wird in der Abbildung 1 gezeigt, daß nach subcutaner Applikation von nur 0,3 µg an der Ratte der Schwellenwert bereits überschritten wird.

Die Erfindung betrifft somit auch Verbindungen der allgemeinen Formel **I** zur Verwendung bei der Behandlung von Estrogenmangelerscheinungen und zur Fertilitätskontrolle bei der Frau.

Die erfindungsgemäßen Verbindungen können in der gleichen Weise wie Ethinylestradiol, welches das am meisten verwendete Estrogen ist, formuliert und eingesetzt werden. Sie werden mit den in der galenischen Pharmazie üblichen Zusätzen, Trägersubstanzen und/oder Geschmackskorrigentien nach an sich bekannten Methoden zu den üblichen Arzneimittelformen verarbeitet. Für die orale Applikation kommen insbesondere Tabletten, Dragees, Kapseln, Pillen, Suspensionen oder Lösungen in Frage. Für die parenterale Applikation kommen insbesondere ölige Lösungen, wie zum Beispiel Sesamöl- oder Rizinusöllösungen, in Frage, die gegebenenfalls zusätzlich noch ein Verdünnungsmittel, wie zum Beispiel Benzylbenzoat oder Benzylalkohol, enthalten können.

Die Wirkstoffkonzentration in den pharmazeutischen Zusammensetzungen ist abhängig von der Applikationsform und dem Anwendungsgebiet. So können zum Beispiel Kapseln oder Tabletten zur Behandlung von Estrogenmangelerscheinungen 0,001 bis 0,05 mg Wirkstoff, ölige Lösungen zur intramuskulären Injektion pro 1 ml etwa 0,01 bis 0,1 mg Wirkstoff und Vaginalsalben etwa 0,1 bis 10 mg pro 100 ml Salbe enthalten. Zur Kontrazeption bei der Frau können die erfindungsgemäßen Estrogene in Kombination mit Gestagenen angewandt werden. Tabletten oder Dragees zur täglichen Einnahme einer Tablette oder eines Dragees sollen vorzugsweise 0,003 bis 0,05 mg des erfindungsgemäßen Estrogens und 0,05 bis 0,5 mg eines Gestagens enthalten.

Die erfindungsgemäßen Verbindungen können bei Estrogenmangelerscheinungen der Frau, wie zum Beispiel Amenorrhoe, Dysmenorrhoe, Sterilität, Endometritis, Kolpitis und klimakterischen Beschwerden und zur Prävention der Osteoporose verwendet werden. Ferner können die Verbindungen als estrogene Komponente in hormonellen Kontrazeptiva (Einphasen- und Mehrphasen- sowie Mehrstufenpräparate) eingesetzt werden. Außerdem sind sie in Verbindung mit anderen Wirkstoffen zur Verwendung in hormontragenden Intrauterinpessaren, implantierbaren Wirkstoffträgern sowie in transdermalen Applikationssystemen geeignet.

Die neuen 14α,16α-Ethano- und 14α,16α-Etheno-estratriene der allgemeinen Formel **I**, 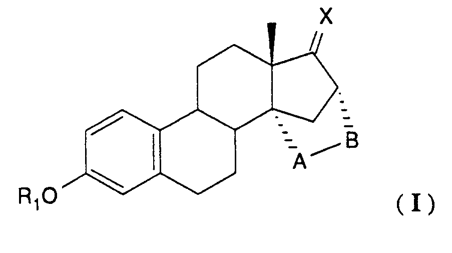
worin
- A - B: eine C-C-Einfach- oder C-C-Doppelbindung,
- R₁: ein Wasserstoffatom, eine Methyl- oder Acylgruppe mit 1 - 12 Kohlenstoffatomen und
- X: Sauerstoff oder 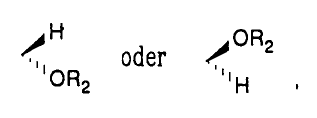 wobei R₂ ein Wasserstoffatom oder eine Acylgruppe mit 1 - 12 Kohlenstoffatomen darstellt,
bedeuten,
werden hergestellt, indem man eine 14α,17α-Ethano- oder 14α,17α-Etheno-16β,17β-dihydroxyverbindung der allgemeinen Formel **II**, 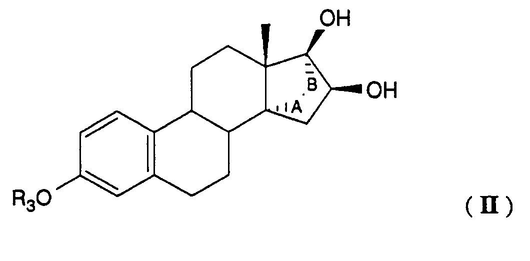
worin
- A - B: eine C-C-Einfach- oder C-C-Doppelbindung und
- R₃: eine Methyl- oder Acylgruppe mit 1 - 12 Kohlenstoffatomen bedeuten,
unter Umlagerung zu Verbindungen der allgemeinen Formel **I** mit X in der Bedeutung von Sauerstoff dehydratisiert, gegebenenfalls anschließend die 17-Ketogruppe zur 17α- oder 17β-Hydroxygruppe reduziert, gewünschtenfalls vor oder nach der Reduktion der 17-Ketogruppe, wenn A - B eine C-C-Doppelbindung bedeutet, diese katalytisch hydriert, gegebenenfalls den 3-Methylether spaltet oder die 17-Hydroxygruppe verestert, gegebenenfalls die 3-Hydroxygruppe partiell oder die 3- und 17-Hydroxygruppen gleichzeitig verestert und gegebenenfalls eine so erhaltene 3,17-Diacyloxyverbindung selektiv zur 3-Hydroxy-17-acyloxyverbindung verseift.

Die Umlagerung der 16β,17β-Dihydroxyverbindungen der allgemeinen Formel **II** zur 17-Ketoverbindungen der allgemeinen Formel **I** mit X in der Bedeutung von Sauerstoff erfolgt unter Dehydratisierungsbedingungen über eine Eliminierung unter Ausbildung eines Kations. Nach einer bevorzugten Ausführungsform werden die Dihydroxyverbindungen mit Methansulfonylchlorid in Pyridin bei Temperaturen von -10 bis +10 °C behandelt, gegebenenfalls unter Zusatz von Schwefeldioxid. Die Umlagerungsprodukte bilden sich in überraschend hoher Ausbeute, wobei in 3-Stellung vorhandene Acyloxygruppen teilweise verseift werden. 3-Hydroxy- und 3-Acyloxyverbindungen können durch einfache Chromatographie getrennt werden. Je nach der letztlich gewünschten Verbindung können die teilweise verseiften Verbindungen auch vollständig verseift oder reacyliert werden.

Die sich gegebenenfalls anschließende Reduktion der 17-Ketogruppe zur 17α-Hydroxygruppe wird vorzugsweise mit einem gemischten Metallhydrid durchgeführt. Als gemischtes Metallhydrid ist besonders Natriumborhydrid geeignet. Als Lösungsmittel werden Alkohole, vorzugsweise Ethanol bei Reaktionstemperaturen von 0 bis 50 °C verwendet.

Sollen Verbindungen der allgemeine Formel **I** mit einer 17β-Hydroxygruppe hergestellt werden, wird die Reduktion der 17-Ketogruppe radikalisch, vorzugsweise mit einem Alkali- oder Erdalkalimetall, insbesondere Lithium in flüssigem Ammoniak, vorgenommen. Die sich gegebenenfalls anschließende Spaltung eines 3-Methylethers wird nach den üblichen Methoden der Steroidetherspaltung vorgenommen. So kann die 3-Methyletherspaltung vorzugsweise mit einer Lewissäure in einem inerten Lösungsmittel in der Siedehitze durchgeführt werden. Als Lewis-Säuren sind beispielsweise Bortrifluoridetherat oder Diisobutylaluminiumhydrid (DIBAH) geeignet. Als Lösungsmittel kommen Benzol, Toluol, Tetrahydrofuran, Dioxan u. a. in Frage.

Die Verseifung der Acyloxygruppen kann in an sich bekannter Weise erfolgen. Beispielsweise wird die Verseifung mit Basen in wäßrig-alkoholischer Lösung, wie Kaliumhydroxid oder Kalium- bzw. Calciumcarbonat in wäßrig-methanolischer Lösung, vorgenommen. Für die sich gegebenenfalls anschließende Veresterung der phenolischen und sekundären Hydroxygruppe kommen die üblicherweise in der Steroidchemie zur Veresterung angewendeten Verfahren in Frage. Beispielsweise sei die Umsetzung mit Essigsäure oder Acetanhydrid in Gegenwart starker Säuren, wie zum Beispiel Trifluoressigsäure, Perchlorsäure oder p-Toluolsulfonsäure, bei Raumtemperatur oder etwas angehobener Temperatur oder die Umsetzung mit Acetanhydrid in Gegenwart von Aminen, vorzugsweise Pyridin, bei etwa 20 - 80 °C genannt. Die Synthesen der beiden möglichen Halbester erfolgen durch partielle Veresterung oder partielle Verseifung:
a) Ausgehend von den 3,17-Dihydroxyverbindungen lassen sich durch selektive Veresterung der phenolischen Hydroxygruppe die 3-Acyloxy-17-hydroxyverbindungen erhalten. Die Reaktionen werden durch Umsetzungen des entsprechenden Säureanhydrids in Gegenwart eines heterocyclischen Stickstoffaromaten, vorzugsweise Pyridin, erreicht. Als Reaktionstemperatur eignet sich der Bereich zwischen Raum- und Siedetemperatur der Reaktionsmischung.
b) Ausgehend von den 3,17-Diacyloxyverbindungen lassen sich durch selektive Verseifung der phenolischen Acyloxygruppe die 3-Hydroxy-17-acyloxyverbindungen erhalten. Die Synthesen erfolgen durch Umsetzungen mit einem Alkalicarbonat oder Erdalkalicarbonat, vorzugsweise Kalium- oder Calciumcarbonat, in wäßrig-methanolischer Lösung. Als Reaktionstemperatur eignet sich der Bereich zwischen Raum- und Siedetemperatur der Reaktionsmischung.

### Beispiel 1

Eine Lösung von 920 mg 3-Acetoxy-14α,17α-ethano-estra-1,3,5(10)-trien-16β,17β-diol in 9.2 ml Pyridin wird unter Eiskühlung mit 1.42 ml Methansulfonylchlorid, das 3 % Schwefeldioxid gelöst enthält, versetzt. Das Gemisch wird 20 min bei 0 °C gerührt und anschließend das Reaktionsprodukt mit Wasser gefällt. Der Niederschlag wird isoliert, mit Wasser gewaschen, getrocknet und an 150 g Kieselgel chromatographiert. Mit 2 l Hexan-Ethylacetat-Gemisch (7:3) eluiert man 400 mg 3-Acetoxy-14α,16α-ethano-estra-1,3,5(10)-trien-17-on, Schmelzpunkt 102 °C, sowie 420 mg 3-Hydroxy-14α,16α-ethano-estra-1,3,5(10)-trien-17-on. Schmelzpunkt 265 °C.

### Beispiel 2

Eine Lösung von 420 mg 3-Hydroxy-14α,16α-ethano-estra-1,3,5(10)-trien-17-on in 30 ml Ethanol wird mit 400 mg Natriumborhydrid versetzt und 3 h bei Raumtemperatur unter Argon gerührt. Das Reaktionsgemisch wird mit Ethylacetat verdünnt, die Lösung mit Natriumchloridlösung gewaschen, über Natriumsulfat getrocknet und im Vakuum eingedampft. Der Rückstand wird an 75 g Kieselgel mit 1 l Hexan-Ethylacetat-Gemisch (4:1) chromatographiert. Es werden 270 mg eluiert, die nach dem Umkristallisieren aus Diethylether-Hexan, 181 mg 14α,16α-Ethano-estra-1,3,5(10)-trien-3,17α-diol ergeben. Schmelzpunkt 179 °C.

### Beispiel 3

Eine Lösung von 500 mg 3-Methoxy-14α,17α-ethano-estra-1,3,5(10)-trien-16β,17β-diol in 5 ml Pyridin wird unter Eiskühlung mit 0.8 ml Methansulfonylchlorid, das 3 % Schwefeldioxid gelöst enthält, versetzt. Das Gemisch wird 20 min bei 0 °C gerührt, anschließend wird das Reaktionsprodukt mit Wasser gefällt. Der Niederschlag wird isoliert, mit Wasser gewaschen, getrocknet und an 150 g Kieselgel chromatographiert. Mit 2 l Pentan-Diethylether-Gemisch (4:1) eluiert man 450 mg kristallines Produkt, das aus Dichlormethan-Hexan umkristallisiert wird. Man erhält 370 mg 3-Methoxy-14α,16α-ethano-estra-1,3,5(10)-trien-17-on. Schmelzpunkt 180 °C.

### Beispiel 4

Eine Lösung von 300 mg 3-Methoxy-14α,16α-ethano-estra-1,3,5(10)-trien-17-on in 25 ml Ethanol wird mit 300 mg Natriumborhydrid versetzt und 4 h bei Raumtemperatur unter Argon gerührt. Das Reaktionsgemisch wird mit Ethylacetat verdünnt, die Lösung mit Natriumchloridlösung gewaschen, über Natriumsulfat getrocknet und im Vakuum eingedampft. Aus dem Rückstand gewinnt man durch Umkristallisieren aus Dichlormethan-Hexan 118 mg 3-Methoxy-14α,16α-ethano-estra-1,3,5(10)-trien-17α-ol. Schmelzpunkt 122 °C.

### Beispiel 5

Eine Lösung von 140 mg 14α,16α-Ethano-estra-1,3,5(10)-trien-3,17α-diol in 6.0 ml Pyridin wird mit 3.0 ml Acetanhydrid versetzt und 10 min bei Raumtemperatur gerührt. Das Reaktionsgemisch wird mit 20 ml Wasser versetzt, das ausgefällte Produkt in Dichlormethan aufgenommen, die Lösung wird getrocknet und im Vakuum eingedampft. Der Rückstand wird aus Diethylether-Pentan kristallisiert und ergibt 108 mg 3-Acetoxy-14α,16α-ethano-estra-1,3,5(10)-trien-17α-ol. Schmelzpunkt 153 °C.

### Beispiel 6

Eine Lösung von 200 mg 14α,16α-Ethano-estra-1,3,5(10)-trien-3,17α-diol in 8.0 ml Pyridin wird mit 4.0 ml Acetanhydrid versetzt und 15 h bei Raumtemperatur gerührt. Das Reaktionsgemisch wird mit 30 ml Wasser versetzt, das ausgefällte Produkt in Dichlormethan aufgenommen, die Lösung wird getrocknet und im Vakuum eingedampft. Der Rückstand, aus Dichlormethan-Hexan kristallisiert, ergibt 120 mg 3,17α-Diacetoxy-14α,16α-ethano-estra-1,3,5(10)-trien. Schmelzpunkt 178 °C.

### Beispiel 7

Eine Lösung von 60 mg 3,17α-Diacetoxy-14α,16α-ethano-estra-1,3,5(10)-trien in 10 ml Methanol wird mit 1.0 ml Wasser sowie 60 mg Calciumcarbonat versetzt und 48 h zum Sieden erhitzt. Anschließend wird die Reaktionsmischung filtriert und und im Vakuum eingedampft. Der kristalline Rückstand von 50 mg wird aus Dichlormethan-Diisopropylether umkristallisiert. Ausbeute 22 mg 17α-Acetoxy-14α,16α-ethano-estra-1,3,5(10)-trien-3-ol. Schmelzpunkt 250 °C.

### Beispiel 8

Eine Lösung von 200 mg 3-Acetoxy-14α,17α-etheno-estra-1,3,5(10)-trien-16β,17β-diol in 2.0 ml Pyridin wird unter Eiskühlung mit 0.3 ml Methansulfonylchlorid, das 3 % Schwefeldioxid gelöst enthält, versetzt. Das Gemisch wird 15 min bei 0 °C gerührt und anschließend das Reaktionsprodukt mit Wasser gefällt. Der Niederschlag wird isoliert, mit Wasser gewaschen, getrocknet und an Kieselgel chromatographiert. Mit einem Pentan-Diethylether-Gemisch (7:3) eluiert man 60 mg, die aus Dichlormethan-Diisopropylether umkristallisiert, 64 mg 3-Acetoxy-14α,16α-etheno-estra-1,3,5(10)-trien-17-on ergeben. Schmelzpunkt 127.8 °C. Weiterhin werden 260 mg eluiert, die aus Dichlormethan-Methanol umkristallisiert, 103 mg 3-Hydroxy-14α,16α-etheno-estra-1,3,5(10)-trien-17-on ergeben. Schmelzpunkt 261.6 °C.

### Beispiel 9

Eine Lösung von 210 mg 3-Hydroxy-14α,16α-etheno-estra-1,3,5(10)-trien-17-on in 30 ml Ethanol wird mit 200 mg Natriumborhydrid versetzt und 3 h bei Raumtemperatur unter Argon gerührt. Das Reaktionsgemisch wird mit Ethylacetat verdünnt, die Lösung mit Natriumchloridlösung gewaschen, über Natriumsulfat getrocknet und im Vakuum eingedampft. Der Rückstand von 190 mg wird aus Dichlormethan-Methanol-Diisopropylether umkristallisiert, ergibt 95 mg 14α,16α-Etheno-estra-1,3,5(10)-trien-3,17α-diol. Schmelzpunkt 249.9 °C.

### Beispiel 10

Eine Lösung von 180 mg 14α,17α-Etheno-estra-1,3,5(10)-trien-3,17α-diol in einem Gemisch aus 10 ml Ethanol und 5 ml Tetrahydrofuran wird mit 50 mg Palladium-Kohle (10 % Pd) versetzt und unter Normaldruck hydriert. Nach 40 min wird vom Katalysator abfiltriert, das Filtrat wird im Vakuum eingedampft, der Rückstand an 100 g Kieselgel mit einem Hexan-Ethylacetat-Gemisch (4:1) chromatographiert. Man eluiert 121 mg kristallines 14α,17α-Ethano-estra-1,3,5(10)-trien-3,17α-diol. Schmelzpunkt 174.2 °C.

### Beispiel 11

Man kondensiert 150 ml Ammoniak unter Kühlung mit festem Kohlendioxid, setzt 2.5 g kleingeschnittenes Lithium hinzu, rührt 15 min und versetzt die tiefblaue Lösung tropfenweise innerhalb von 20 min mit einer Lösung von 2.4 g 3-Hydroxy-14α,16α-ethano-estra-1,3,5(10)-trien-17-on in 150 ml wasserfreiem Tetrahydrofuran und rührt weitere 20 min. Anschließend wird vorsichtig eine gesättigte Ammoniumchloridlösung hinzugefügt und die Mischung auf Raumtemperatur gebracht. Nach dem abdampfen des Ammoniaks wird mit 150 ml Wasser versetzt und mehrfach mit Dichlormethan-Methanol (9:1) extrahiert. Die vereinigten Extrakte werden über Natriumsulfat getrocknet und im Vakuum eingedampft. Der Rückstand wird an Kieselgel chromatographiert. Man eluiert mit einem Dichlormethan-Ethylacetat-Gemisch (75:25) 1.1 g kristallines Rohprodukt, das nach dem Umkristallisieren aus Dichlormethan-Diisopropylether 496 mg 14α,16α-Ethano-estra-1,3,5(10)-trien-3,17β-diol ergibt. Schmelzpunkt 148 °C.

### Herstellung der Ausgangsverbindungen

### 3,16β,17β-Triacetoxy-14α,17α-etheno-estra-1,3,5(10)-trien-16α-carbonitril

Eine Lösung von 3.0 g Estra-1,3,5(10),14,16-pentaen-3,17-diol-diacetat in 10 ml Dichlormethan wird mit 10 ml 1-Cyano-vinylacetat versetzt und 4 Tage im geschlossenem Rohr auf 140 °C erhitzt. Die verharzte Reaktionsmischung wird im Mörser zerkleinert und mit siedendem Aceton behandelt. Nach dem Dekantieren und Verdampfen des Lösungsmittels verbleiben 4.9 g eines Rückstands, der an Kieselgel chromatographiert wird. Man eluiert mit einem Hexan-Ethylacetat-Gemisch (7:3) und erhält 3.78 g, die aus Dichlormethan-Hexan umkristallisiert, 3.28 g 3,16β,17β-Triacetoxy-14α,17α-etheno-estra-1,3,5(10)-trien-16α-carbonitril ergeben. Schmelzpunkt 162 °C.

### 14α,17α-Etheno-estra-1,3,5(10)-trien-3,16β,17β-triol

Eine Lösung von 6.3 g 3,16β,17β-Triacetoxy-14α,17α-etheno-estra-1,3,5(10)-trien-16α-carbonitril in 180 ml wasserfreiem Ethanol wird mit 6.0 g Natriumborhydrid versetzt und 15 h bei Raumtemperatur unter Argon gerührt. Das Reaktionsgemisch wird mit Ethylacetat verdünnt, die Lösung mit Wasser gewaschen, über Natriumsulfat getrocknet und im Vakuum eingedampft. Der Rückstand wird an 200 g Kieselgel mit 3 l Dichlormethan-Methanol-Gemisch (95:5) chromatographiert. Es werden 3.21 g einer kristallinen Substanz eluiert. Nach dem Umkristallisieren aus Diisopropylether-Methanol erhält man 2.38 g 14α,17α-Etheno-estra-1,3,5(10)-trien-3,16β,17β-triol. Schmelzpunkt 227 °C.

### 14α,17α-Ethano-estra-1,3,5(10)-trien-3,16β,17β-triol

Eine Lösung von 110 mg 14α,17α-Etheno-estra-1,3,5(10)trien-3,16β,17β-triol in einem Gemisch aus 6 ml Ethanol und 1.5 ml Tetrahydrofuran wird mit 20 mg Palladium-Kohle (10% Pd) versetzt und unter Normaldruck hydriert. Nach Aufnahme von 10.5ml Wasserstoff (ber. 8.7 ml) innnerhalb von 30 min wird der Palladium-Katalysator abfiltriert. Das Filtrat wird im Vakuum eingedampft und der Rückstand aus einem Methanol-Diisopropylether-Gemisch umkristallisiert. Ausbeute: 105 mg 14α,17α-Ethano-estra-1,3,5(10)-trien-3,16β,17β-triol. Schmelzpunkt 230 °C.

### 3-Acetoxy-14α,17α-etheno-estra-1,3,5(10)trien-16β,17β-diol

Ein Lösung von 2.36 g 14α,17α-Etheno-estra-1,3,5(10)-trien-3,16β,17β-triol in einem Gemisch aus 20 ml Pyridin und 5 ml Acetanhydrid wird 20 min bei Raumtemperatur gehalten. Die Reaktionslösung wird in Eis/Kochsalzlösung eingerührt, der ausgefallenen Niederschlag wird abfiltriert, mit Wasser gewaschen getrocknet und an 125 g Kielselgel chromatographiert. Mit 5 l Hexan-Ethylacetat-Gemisch (7:3) eluiert man 1.79 g kristallines 3-Acetoxy-14α,17α-etheno-estra-1,3,5(10)-trien-16β,17β-diol. Schmelzpunkt 202 °C.

### 3-Acetoxy-14α,17α-ethano-estra-1,3,5(10)-trien-16β,17β-diol

Eine Lösung von 1.0 g 3-Acetoxy-14α,17α-etheno-estra-1,3,5(10)-trien-16β,17β-diol in einem Gemisch aus 60 ml Ethanol und 25 ml Tetrahydrofuran wird mit 250 mg Palladium-Kohle (10 % Pd) versetzt und unter Normaldruck hydriert. Nach Aufnahme von 85 ml Wasserstoff (ber. 68 ml) innnerhalb von 5 min wird vom Katalysator abfiltriert. Das Filtrat wird im Vakuum eingedampft und der Rückstand aus Dichlormethan-Diisopropylether umkristallisiert. Man erhält 571 mg 3-Acetoxy-14α,17α-ethano-estra-1,3,5(10)-trien-16β,17β-diol. Schmelzpunkt 193 °C.

### 16β,17β-Diacetoxy-3-methoxy-14α,17α-etheno-estra-1,3,5(10)-trien-16α-carbonitril

Eine Lösung von 2.0 g 3-Methoxy-estra-1,3,5(10),14,16-pentaen-17-ol-acetat in 20 ml Benzol wird mit 4.6 ml 1-Cyano-vinylacetat versetzt und im Druckgefäß 4 Tage auf 100 °C erhitzt. Die Reaktionsmischung wird an 400 g Kieselgel mit Ethylacetat-Hexan (1:1) chromatographiert und ergibt, nach dem Umkristallisieren aus Diisopropylether-Hexan, 1.9 g 16β,17β-Diacetoxy-3-methoxy-14α,17α-etheno-estra-1,3,5(10)-trien-16α-carbonitril. Schmelzpunkt 144 °C.

### 3-Methoxy-14α,17α-etheno-estra-1,3,5(10)-trien-16β,17β-diol

Eine Lösung von 400 mg 16β,17β-Diacetoxy-3-methoxy-14α,17α-etheno-estra-1,3,5(10)-trien-16α-carbonitril in 30 ml wasserfreiem Ethanol wird mit 300 mg Natriumborhydrid versetzt und 3 h bei Raumtemperatur unter Argon gerührt. Das Reaktionsgemisch wird mit Ethylacetat verdünnt, die Lösung mit Wasser gewaschen, über Natriumsulfat getrocknet und im Vakuum eingedampft. Der Rückstand wird an 100g Kieselgel mit einem Hexan-Ethylacetat-Gemisch (0-20 % Ethylacetat) chromatographiert. Es werden 151 mg eluiert, die nach dem Umkristallisieren aus Dichlormethan-Methanol 111 mg 3-Methoxy-14α,17α-etheno-estra-1,3,5(10)-trien-16β,17β-diol ergeben. Schmelzpunkt 167 °C.

### 3-Methoxy-14α,17α-ethano-estra-1,3,5(10)-trien-16β,17β-diol

Eine Lösung von 700 mg 3-Methoxy-14α,17α-etheno-estra-1,3,5(10)-trien-16β,17β-diol in einem Gemisch aus 30 ml Ethanol und 15 ml Tetrahydrofuran wird mit 200 mg Palladium-Kohle (10 % Pd) versetzt und unter Normaldruck hydriert. Nach Aufnahme von 57 ml Wasserstoff (berechnet 51 ml) innnerhalb von 40 min wird vom Katalysator abfiltriert. Das Filtrat wird im Vakuum eingedampft, der Rückstand an 100 g Kieselgel mit einem Hexan-Ethylacetat-Gemisch (7:3) chromatographiert und die eluierte Substanz von 540 mg aus Dichlormethan-Diisopropylether umkristallisiert. Man erhält 268 mg 3-Methoxy-14α,17α-ethano-estra-1,3,5(10)-trien-16β,17β-diol. Schmelzpunkt 172 °C.

## Patentansprüche

1. 14α,16α-Ethano- und 14α,16α-Etheno-estratriene der allgemeinen Formel **I**, 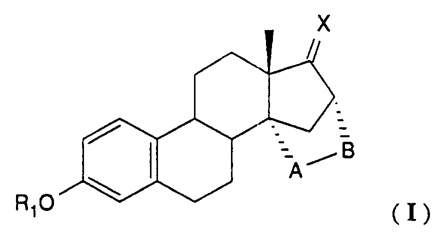 worin
A - B eine C-C-Einfach- oder C-C-Doppelbindung,
R₁ ein Wasserstoffatom, eine Methyl- oder Acylgruppe mit 1 - 12 Kohlenstoffatomen und
X Sauerstoff oder 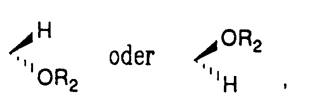 wobei R₂ ein Wasserstoffatom oder eine Acylgruppe mit 1 - 12 Kohlenstoffatomen darstellt,
bedeuten,

2. Verbindungen nach Anspruch 1:
3-Acetoxy-14α,16α-ethano-estra-1,3,5(10)-trien-17-on
3-Acetoxy-14α,16α-etheno-estra-1,3,5(10)-trien-17-on
3-Hydroxy-14α,16α-ethano-estra-1,3,5(10)-trien-17-on
3-Hydroxy-14α,16α-etheno-estra-1,3,5(10)-trien-17-on
14α,16α-Ethano-estra-1,3,5(10)-trien-3,17α-diol
14α,16α-Etheno-estra-1,3,5(10)-trien-3,17α-diol
14α,16α-Ethano-estra-1,3,5(10)-trien-3,17β-diol
14α,16α-Etheno-estra-1,3,5(10)-trien-3,17β-diol
3-Methoxy-14α,16α-ethano-estra-1,3,5(10)-trien-17-on
3-Methoxy-14α,16α-etheno-estra-1,3,5(10)-trien-17-on
3-Methoxy-14α,16α-ethano-estra-1,3,5(10)-trien-17α-ol
3-Methoxy-14α,16α-etheno-estra-1,3,5(10)-trien-17α-ol
3-Methoxy-14α,16α-ethano-estra-1,3,5(10)-trien-17β-ol
3-Methoxy-14α,16α-etheno-estra-1,3,5(10)-trien-17β-ol
3-Acetoxy-14α,16α-ethano-estra-1,3,5(10)-trien-17α-ol
3-Acetoxy-14α,16α-etheno-estra-1,3,5(10)-trien-17α-ol
3-Acetoxy-14α,16α-ethano-estra-1,3,5(10)-trien-17β-ol
3-Acetoxy-14α,16α-etheno-estra-1,3,5(10)-trien-17β-ol
3,17α-Diacetoxy-14α,16α-ethano-estra-1,3,5(10)-trien
3,17α-Diacetoxy-14α,16α-etheno-estra-1,3,5(10)-trien
3,17β-Diacetoxy-14α,16α-ethano-estra-1,3,5(10)-trien
3,17β-Diacetoxy-14α,16α-etheno-estra-1,3,5(10)-trien
17α-Acetoxy-14α,16α-ethano-estra-1,3,5(10)-trien-3-ol
17α-Acetoxy-14α,16α-etheno-estra-1,3,5(10)-trien-3-ol
17β-Acetoxy-14α,16α-ethano-estra-1,3,5(10)-trien-3-ol
17β-Acetoxy-14α,16α-etheno-estra-1,3,5(10)-trien-3-ol

3. Pharmazeutische Präparate dadurch gekennzeichnet, daß sie eine Verbindung gemäß Ansprüche 1 - 2 sowie einen pharmakologisch verträglichen Träger enthalten

4. Verwendung einer Verbindung gemäß Ansprüche 1 - 2 zur Herstellung von pharmazeutischen Präparaten zur Behandlung von Estrogenmangelerscheinungen und zur Fertilitätskontrolle bei der Frau.

5. Verfahren zur Herstellung von 14α,16α-Ethano- und 14α,16α-Etheno-estra trienen der allgemeinen Formel **I**, 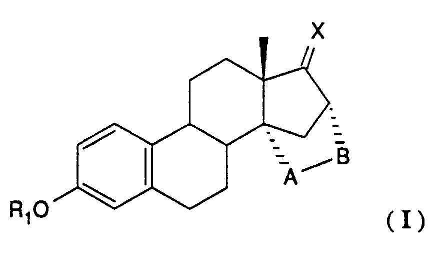 worin
A - B eine C-C-Einfach- oder C-C-Doppelbindung,
R₁ ein Wasserstoffatom, eine Methyl- oder Acylgruppe mit 1 - 12 Kohlenstoffatomen und
X Sauerstoff oder 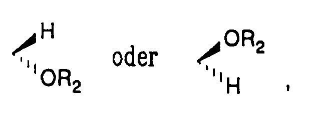 wobei R₂ ein Wasserstoffatom oder eine Acylgruppe mit 1 - 12 Kohlenstoffatomen darstellt,
bedeuten,
dadurch gekennzeichnet, daß man eine 14α,17α-Ethano- oder 14α,17α-Etheno-16β,17β-dihydroxyverbindung der allgemeinen Formel **II**, 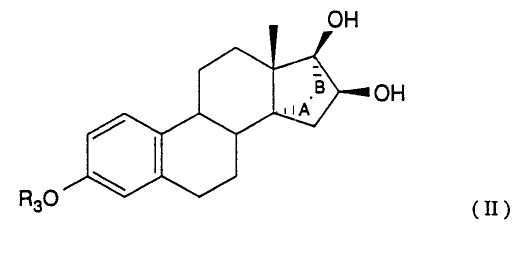 worin
A - B eine C-C-Einfach- oder C-C-Doppelbindung und
R₃ eine Methyl- oder Acylgruppe mit 1 - 12 Kohlenstoffatomen bedeuten,
unter Umlagerung zu Verbindungen der allgemeinen Formel **I** mit X in der Bedeutung von Sauerstoff dehydratisiert, gegebenenfalls anschließend die 17-Ketogruppe zur 17α- oder 17β-Hydroxygruppe reduziert, gewünschtenfalls vor oder nach der Reduktion der 17-Ketogruppe, wenn A - B eine C-C-Doppelbindung bedeutet, diese katalytisch hydriert, gegebenenfalls den 3-Methylether spaltet oder die 17-Hydroxygruppe verestert, gegebenenfalls die 3-Hydroxygruppe partiell oder die 3- und 17-Hydroxygruppen gleichzeitig verestert und gegebenenfalls eine so erhaltene 3,17-Diacyloxyverbindung selektiv zur 3-Hydroxy-17-acyloxyverbindung verseift.

## Claims

1. 14α,16α-ethano- and 14α,16α-etheno-oestratrienes of the general formula I 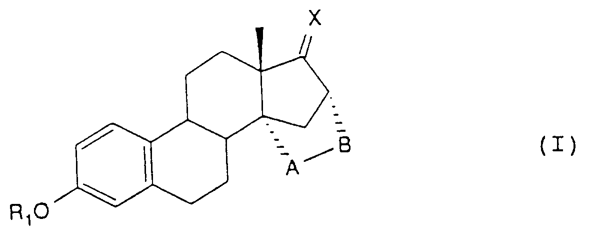 wherein
A - B represents a C-C single bond or a C-C double bond,
R₁ represents a hydrogen atom, or a methyl or acyl group having from 1 to 12 carbon atoms, and
X represents oxygen or 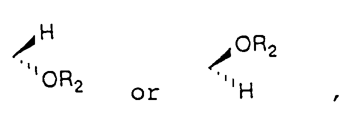 wherein R₂ is a hydrogen atom or an acyl group having from 1 to 12 carbon atoms.

2. Compounds according to claim 1:
3-acetoxy-14α,16α-ethano-oestra-1,3,5(10)-trien-17-one
3-acetoxy-14α,16α-etheno-oestra-1,3,5(10)-trien-17-one
3-hydroxy-14α,16α-ethano-oestra-1,3,5(10)-trien-17-one
3-hydroxy-14α,16α-etheno-oestra-1,3,5(10)-trien-17-one
14α,16α-ethano-oestra-1,3,5(10)-triene-3,17α-diol
14α,16α-etheno-oestra-1,3,5(10)-triene-3,17α-diol
14α,16α-ethano-oestra-1,3,5(10)-triene-3,17β-diol
14α,16α-etheno-oestra-1,3,5(10)-triene-3,17β-diol
3-methoxy-14α,16α-ethano-oestra-1,3,5(10)-trien-17-one
3-methoxy-14α,16α-etheno-oestra-1,3,5(10)-trien-17-one
3-methoxy-14α,16α-ethano-oestra-1,3,5(10)-trien-17α-ol
3-methoxy-14α,16α-etheno-oestra-1,3,5(10)-trien-17α-ol
3-methoxy-14α,16α-ethano-oestra-1,3,5(10)-trien-17β-ol
3-methoxy-14α,16α-etheno-oestra-1,3,5(10)-trien-17β-ol
3-acetoxy-14α,16α-ethano-oestra-1,3,5(10)-trien-17α-ol
3-acetoxy-14α,16α-etheno-oestra-1,3,5(10)-trien-17α-ol
3-acetoxy-14α,16α-ethano-oestra-1,3,5(10)-trien-17β-ol
3-acetoxy-14α,16α-etheno-oestra-1,3,5(10)-trien-17β-ol
3,17α-diacetoxy-14α,16α-ethano-oestra-1,3,5(10)-triene
3,17α-diacetoxy-14α,16α-etheno-oestra-1,3,5(10)-triene
3,17β-diacetoxy-14α,16α-ethano-oestra-1,3,5(10)-triene
3,17β-diacetoxy-14α,16α-etheno-oestra-1,3,5(10)-triene
17α-acetoxy-14α,16α-ethano-oestra-1,3,5(10)-trien-3-ol
17α-acetoxy-14α,16α-etheno-oestra-1,3,5(10)-trien-3-ol
17β-acetoxy-14α,16α-ethano-oestra-1,3,5(10)-trien-3-ol
17β-acetoxy-14α,16α-etheno-oestra-1,3,5(10)-trien-3-ol

3. Pharmaceutical preparations characterised in that they comprise a compound according to claim 1 or claim 2 and a pharmacologically tolerable carrier.

4. Use of a compound according to claim 1 or claim 2 for the manufacture of pharmaceutical preparations for the treatment of oestrogen-deficiency symptoms and for fertility control in women.

5. Process for the preparation of 14α,16α-ethano- and 14α,16α-etheno-oestratrienes of the general formula I 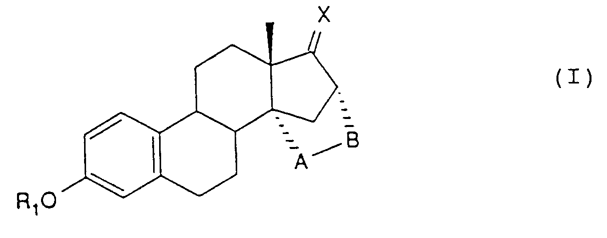 wherein
A - B represents a C-C single bond or a C-C double bond,
R₁ represents a hydrogen atom, or a methyl or acyl group having from 1 to 12 carbon atoms, and
X represents oxygen or 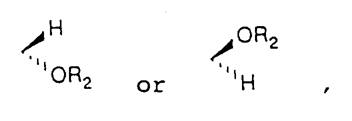 wherein R₂ is a hydrogen atom or an acyl group having from 1 to 12 carbon atoms,
which process is characterised in that a 14α,17α-ethano- or 14α,17α-etheno-16β,17β-dihydroxy compound of the general formula II 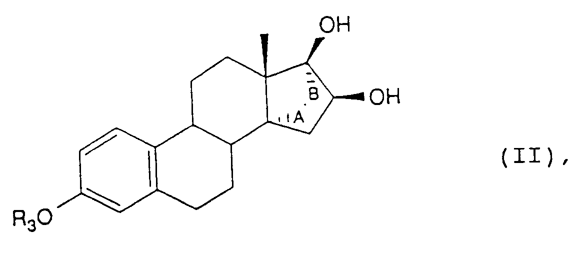 wherein
A - B represents a C-C single bond or a C-C double bond, and
R₃ represents a methyl or acyl group having from 1 to 12 carbon atoms,
is dehydrated with rearrangement to form compounds of the general formula I wherein X is oxygen, optionally the 17-keto group is then reduced to form the 17α- or 17β-hydroxy group, if desired before or after reduction of the 17-keto group, when A - B is a C-C double bond, the latter is catalytically hydrogenated, optionally the 3-methyl ether is cleaved or the 17-hydroxy group is esterified, optionally the 3-hydroxy group is partially esterified or the 3- and 17-hydroxy groups are simultaneously esterified and optionally a resulting 3,17-diacyloxy compound is hydrolysed selectively to form the 3-hydroxy-17-acyloxy compound.

## Revendications

1. 14α,16α-Ethano et 14α,16α-éthèno-estratriène de formule générale I, 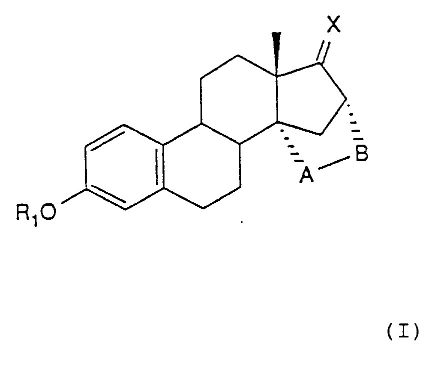 dans laquelle
A-B représente une liaison simple carbone-carbone ou bien une liaison double carbone-carbone,
R₁ signifie un atome d'hydrogène, un groupe méthyle ou acyle ayant de 1 à 12 atomes de carbone et
X signifie l'oxygène ou bien 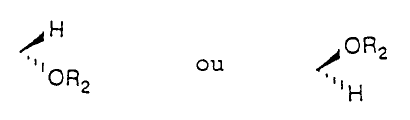 R₂ représentant un atome d'hydrogène ou bien un groupe acyle ayant de 1 à 12 atomes de carbone.

2. Composés selon la revendication 1 :
3-acétoxy-14α,16α-éthano-estra-1,3,5(10)-triène-17-one
3-acétoxy-14α,16α-éthèno-estra-1,3,5(10)-triène-17-one
3-hydroxy-14α,16α-éthano-estra-1,3,5(10)-triène-17-one
3-hydroxy-14α,16α-éthèno-estra-1,3,5(10)-triène-17-one
14α,16α-éthano-estra-1,3,5(10)-triène-3,17α-diol
14α,16α-éthèno-estra-1,3,5(10)-triène-3,17α-diol
14α,16α-éthano-estra-1,3,5(10)-triène-3,17β-diol
14α,16α-éthèno-estra-1,3,5(10)-triène-3,17β-diol
3-méthoxy-14α,16α-éthano-estra-1,3,5(10)-triène-17-one
3-méthoxy-14α,16α-étheno-estra-1,3,5(10)-triène-17-one
3-méthoxy-14α,16α-éthano-estra-1,3,5(10)-triène-17α-ol
3-méthoxy-14α,16α-éthèno-estra-1,3,5(10)-triène-17α-ol
3-méthoxy-14α,16α-éthano-estra-1,3,5(10)-triène-17β-ol
3-méthoxy-14α,16α-éthèno-estra-1,3,5(10)-triène-17β-ol
3-acétoxy-14α,16α-éthano-estra-1,3,5(10)-triène-17α-ol
3-acétoxy-14α,16α-éthèno-estra-1,3,5(10)-triène-17α-ol
3-acétoxy-14α,16α-éthano-estra-1,3,5(10)-triène-17β-ol
3-acétoxy-14α,16α-éthèno-estra-1,3,5(10)-triène-17β-ol
3,17α-diacétoxy-14α,16α-éthano-estra-1,3,5(10)-triène
3,17α-diacétoxy-14α,16α-éthèno-estra-1,3,5(10)-triène
3,17β-diacétoxy-14α,16α-éthano-estra-1,3,5(10)-triène
3,17β-diacétoxy-14α,16α-éthèno-estra-1,3,5(10)-triène
17α-acétoxy-14α,16α-éthano-estra-1,3,5(10)-triène-3-ol
17α-acétoxy-14α,16α-éthèno-estra-1,3,5(10)-triène-3-ol
17β-acétoxy-14α,16α-éthano-estra-1,3,5(10)-triène-3-ol
17β-acétoxy-14α,16α-éthèno-estra-1,3,5(10)-triène-3-ol.

3. Préparations pharmaceutiques caractérisées en ce qu'elles contiennent un composé selon les revendications 1-2 ainsi qu'un support ou vecteur compatible du point de vue pharmacologique.

4. Utilisation d'un composé selon les revendications 1-2 en vue de la préparation de préparations pharmaceutiques pour le traitement de désordres dûs à une carence en oestrogène et pour le contrôle de la fertilité chez la femme.

5. Procéde de préparation de 14α, 16α-éthano- et 14α,16α-éthèno-estra-triènes de formule générale I, 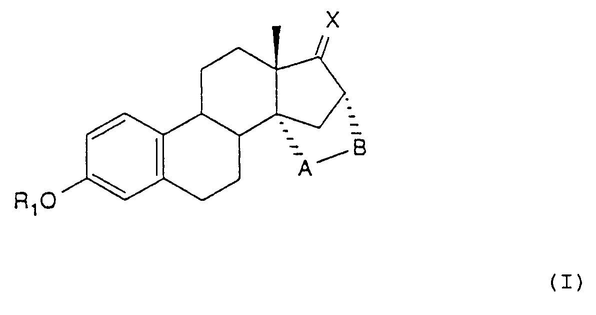 dans laquelle
A - B signifie une liaison simple carbone-carbone ou bien une liaison double carbone-carbone,
R₁ signifie un atome d'hydrogène, un groupe méthyle ou acyle ayant de 1 à 12 atomes de carbone et
X signifie l'oxygène ou bien 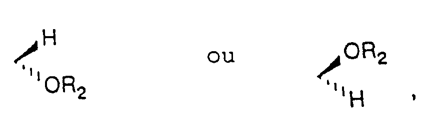 R₂ représentant un atome d'hydrogène ou bien un groupe acyle ayant de 1 à 12 atomes de carbone,
caractérisé en qu'on déshydrate un composé 14α,17α-éthano-ou 14α,17α-éthèno-16β,17β-dihydroxy de formule générale II, 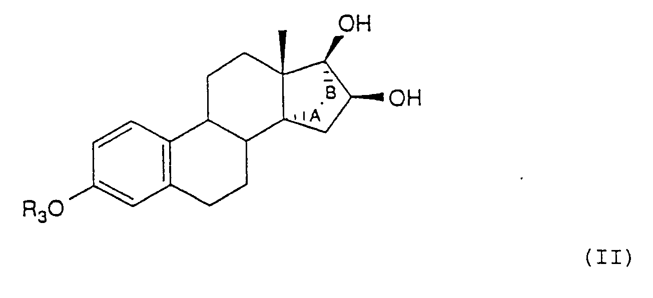 dans laquelle
A - B signifie une liaison simple carbone-carbone ou bien une liaison double carbone-carbone et
R₃ signifie un groupe méthyle ou acyle ayant de 1 à 12 atomes de carbone,
pour donner par transposition des composés de formule générale I avec X signifiant l'oxygène, on réduit ensuite éventuellement le groupe 17-céto en groupe 17α- ou bien 17β-hydroxy, on hydrogène de manière catalytique, dans le cas ou A-B signifie une double liaison carbone-carbone, cette liaison, si on le souhaite, avant ou après la réduction du groupe 17-céto, on clive éventuellement l'éther méthylique en position 3 ou bien on estérifie le groupe hydroxy en position 17, on estérifie éventuellement partiellement le groupe hydroxy en position 3 ou bien simultanément les groupes hydroxy en positions 3 et 17 et on saponifie éventuellement de manière sélective un composé 3,17-diacyloxy ainsi obtenu en composé 3-hydroxy-17-acyloxy.
